# EUROPEAN PATENT APPLICATION

(11) **EP 3 235 510 A1**
(43) Date of publication of application: **25.10.2017**
(21) Application number: 16305450.5
(22) Date of filing: 18.04.2016
(51) Int. Cl.: A61K 45/06, A61K 31/353, A61K 33/04, A61K 33/06, A61K 33/24, A61K 33/30, A61K 33/32, A61K 33/34, A61K 36/54, A23L 33/16, A61P 3/02, A61P 3/10, A61P 3/04

(54) **NUTRITIONAL COMPOSITIONS FOR THE MANAGEMENT OF GLUCOSE METABOLISM**

(71) Applicant: Dialpha, 34980 Montferrier-sur-Lez (FR)
(72) Inventor: BEEJMOHUN, Vickram, 34000 MONTPELLIER (FR); MAZOLLIER, Aude, 34800 CLERMONT L'HERAULT (FR); CHAPAL, Nicolas, 34980 COMBAILLAUX (FR)
(74) Representative: Potter Clarkson LLP

(57) **Abstract**

The present invention relates nutritional compositions that can help to reduce glucose peaks in the blood of a subject after consumption of food with a high glycemic index as well as compositions for treatment or prevention of pre-diabetes, diabetes, hyperinsulinemia, hyperglycemia, reduction of glycemic response and metabolic syndrome. The present invention is a composition comprising a synergistic amount of both procyanidins and minerals.

## Description

The present invention relates generally to the field of food products and nutrional compositions. For example, the present invention aims at natural compounds that can help to reduce glucose peaks in the blood of a subject after consumption of food with a high glycemic index. One embodiment of the present invention is a composition comprising a synergistic amount of both procyanidins and minerals.

The present invention generally relates to methods and compositions for treatment or prevention of pre-diabetes, diabetes, hyperinsulinemia, hyperglycemia, reduction of glycemic response and metabolic syndrome. More specifically, the present disclosure relates to compositions that comprise a synergistic amount of both procyanidins and minerals.

More specifically, the present disclosure relates to administering an amount of procyanidins that is suitable for oral consumption and, in combination with mineral mix comprising source of chromium, source of zinc, source of selenium, source of magnesium, source of manganese and source of copper improves one or more of insulin sensitivity, glucose tolerance, reduces hyperglycemia, fasting hyperglycemia, postprandial hyperglycemia.

Insulin resistance is associated with a number of disease states and conditions and is present in approximately 30-40% of non-diabetic individuals. These disease states and conditions include, but are not limited to, pre-diabetes and metabolic syndrome (also referred to as insulin resistance syndrome). Pre-diabetes is a state of abnormal glucose tolerance characterized by either impaired glucose tolerance (IGT) or impaired fasting glucose (IFG), hyperglycemia such as fasting hyperglycemia and/or postprandial hyperglycemia. Patients with pre-diabetes are insulin resistant and are at high risk for future progression to overt type 2 diabetes. Metabolic syndrome is an associated cluster of traits that include, but is not limited to, hyperglycemia, fasting hyperglycemia, postprandial hyperglycemia, hyperinsulinemia, abnormal glucose tolerance, obesity, redistribution of fat to the abdominal or upper body compartment, hypertension, dysfibrinolysis and a dyslipidemia characterized by high triglycerides, low HDL-cholesterol and high LDL-cholesterol. Insulin resistance has been linked to each of the traits, suggesting metabolic syndrome and insulin resistance are intimately related to one another.

During the past decades, the prevalence of obesity has increased worldwide to epidemic proportion. Approximately 2 billion of people worldwide are overweight or obese, conditions that increase mortality, mobility and economical costs. Obesity develops when energy intake is greater than energy expenditure, the excess energy being stored mainly as fat in adipose tissue. Body weight loss and prevention of weight gain can be achieved by reducing energy intake or bioavailability, increasing energy expenditure and/or reducing storage as fat.

Another condition adversely affecting some individuals is that their body tissues do not respond properly to insulin. Insulin receptors in the tissues cease to function adequately and gluco-dependent cells fail to recognize the presence of insulin. As a result, the pancreas needs to secrete more insulin to help glucose enter these cells. The pancreas tries to keep up with this increased demand for insulin by producing more. This phenomenon is called insulin resistance (also known as low insulin sensitivity). Many people with insulin resistance have high levels of both glucose and insulin circulating in their blood at the same time. Eventually, the pancreas fails to keep up with the body's need for insulin, leading to type 2 diabetes.

Insulin resistance and type 2 diabetes are associated with increased risk of heart attacks, strokes, amputation, diabetic retinopathy and kidney failure. For extreme cases, circulation of limbs is affected, potentially requiring amputation. Loss of hearing, eyesight and cognitive ability has also been linked to these conditions.

At some point a loss of control of blood glucose begins to emerge, resulting in impaired glucose tolerance (IGT) or impaired fasting glucose (IFG) and may ultimately result in type 2 diabetes. Therefore IGT and IFG refer to metabolic states intermediate between normal glucose homeostasis and diabetes.

A further test, the oral glucose tolerance test (OGTT), may be performed to assess whether the patient is diabetic or has IGT. The OGTT consists of a glucose drink containing 75 g of glucose. The patient's blood sugar level is measured at one and two hours following administration of the drink. As glucose is an essential nutrient for the human body, its circulating levels must be carefully maintained constant, in order to supply adequate amounts to peripheral tissues. The liver plays a central role in glucose homeostasis by balancing its uptake and storage via glycogenesis and its release via glycogenolysis and gluconeogenesis. An impairment of glucose homeostasis is a typical feature of type 2 diabetes. Patients with type 2 diabetes exhibit increased hepatic glucose production (HGP), which is identified as the main cause of fasting hyperglycemia and is associated with a reduced plasma glucose clearance (Gastaldelli et al., Diabetes 2000, 49:1367-1373) and a 25-45% reduced synthesis of glycogen compared with non-diabetic subjects (Roden et al., Best Pract Res Clin Endocrinol Metab 2003, 17:365-83). Limiting blood glucose peaks after a meal, in particular in diabetic subjects, constitutes an important target of the overall glycemic control strategy. Management of insulin resistance in children and adults is essentially based on dietary and lifestyle changes, including healthier dietary habits and increased exercise. These practices can be very efficient in improving insulin sensitivity and in slowing the progression of the disease, but they are difficult to apply and actually not followed by most patients. Type 2 diabetes can be treated with drugs promoting insulin sensitivity, but their efficacy in reducing the rate of progression of the disease is quite low. Insulin treatment is required during the most advanced phases of the disease.

Products containing n-3 polyunsaturated fatty acids, fibers, oligosaccharides and even probiotics have been proposed as nutritional solutions to improve insulin sensitivity and to reduce insulin resistance. However, the efficacy of these nutritional interventions is quite marginal and even controversial, with studies showing no or even deleterious effects.

Dietary minerals intake may have an effect on metabolic syndrome, especially in the treatment of postprandial and fasting hyperglycemia. A recent review of Siddiqui et al. concluded that macro and trace elements play an important role in glucose metabolism and insulin resistance in type 2 diabetes (Siddiqui et al., ScientificWorldJournal 2014, 2014:461591). The proposed mechanism of trace elements enhancing insulin action includes activation of insulin receptor sites (kinase activation), serving as cofactors or components of enzyme systems involved in glucose metabolism.

A systematic review and meta-analysis of studies have evaluated the beneficial effects of oral zinc supplementation on fasting blood glucose, 2-hour postprandial blood glucose, cholesterol and lipid parameters in patients with type 2 diabetes. These data showed several beneficial metabolic and clinical effects due to zinc supplementation in patients with diabetes mellitus, namely improved glycemic control and lipid parameters. This zinc supplementation causes significant reduction of fasting blood glucose, postprandial blood glucose and HbA1c in patients with type 2 diabetes. In-vivo and in-vitro studies have demonstrated the insulin mimetic and hypoglycemic properties of zinc complexes (Jayawaderna et al., Diabetol Metab Syndr 2012, 4:13).

Systematic reviews and meta-analysis showed an existing relation between intakes of dietary magnesium and metabolic syndrome and related diseases, namely insulin resistance, diabetes mellitus, hypertension, dyslipidemia and cardiovascular diseases. Indeed, magnesium supplementation seems to have beneficial effect in diabetic patients (Sarrafzadegan et al., Nutrition 2016, 32:409-17; Sales et al., Clin Nutr 2011, 30:359-64). Another review, Guerrero-Romero et al. showed a beneficial effect of oral supplementation with magnesium chloride on fasting and postprandial glucose levels and insulin sensitivity (Guerrero-Romero et al., Arch Med Res 2005, 36:250-7).

Holger Steinbrenner summarized evidence for an interference of dietary selenium supplementation with insulin-regulated molecular pathways, as an insulin-mimetic micronutrient (Steinbrenner, Free Radic Biol Med 2013, 65:1538-47). Recent epidemiological and interventional studies revealed a surprising association between high plasma selenium levels and type 2 diabetes, hyperglycemia and dyslipidemia. Another review, by Tajaddini et al., summarized the information about the relationship between metabolic syndrome components and selenium as trace element. However, the author concluded that large controversies exist about the effects of selenium on metabolic syndrome, which may be due to differences in study design and population (Tajaddini et al., J Res Med Sci 2015, 20:782-9).

In the review of Guerrero-Romero et al., chromium is an essential mineral and its supplementation is involved in the action of insulin and energetic metabolism, without serious adverse effects. It has been also reported that chromium improves the action of insulin by improving tyrosine kinase activity on the insulin receptor and decreases the hepatic extraction of plasma insulin leading to a more efficient glucose uptake (Guerrero-Romero et al., Arch Med Res 2005, 36:250-7).

Balk et al. reviewed the effect of chromium supplementation on glucose metabolism and lipids levels. They observed no significant effect of chromium supplementation on lipid or glucose metabolism in healthy people, but glycemia is improved in patients with diabetes (Balk et al., Diabetes Care 2007, 30:2154-63). Frauchiger et al. studied the acute effect of chromium supplementation on postprandial glycemia in healthy young men taking a test meal containing 75 g of available carbohydrates. They concluded that an acutely administered single dose of chromium (400 or 800 µg) improved glycemia after a high-glycemic meal in about 80% of young healthy subjects, without visible effects on insulin concentration. These results seem to support the potentiating role of chromium on insulin action. However, additional studies are required to examine further the effects of acute chromium supplementation in humans (Frauchiger et al., J Am Coll Nutr 2004, 23:351-7).

Manganese is a trace mineral which plays a role in fat and carbohydrate metabolism, calcium absorption and blood sugar regulation in human. Recent studies have suggested that micronutrients such as magnesium, manganese and copper have beneficial effects on metabolic syndrome related disorders. The relationship between the intakes of these minerals and the metabolic syndrome risks was analyzed. In the men, daily intakes of magnesium and copper were significantly lower in the group with metabolic syndrome compared to the control group and, in the women, daily intakes of magnesium, manganese and copper were significantly lower in the group with metabolic syndrome (Choi and Bae, Biol Trace Elem Res 2013, 156:56-66).

It thus appears that the effects of minerals on glucose control is highly variable, specific and dependent of the mineral and the form of mineral used and there is no real consensus about the exact role of use of minerals in glycemia control.

According to literature, high polymeric proanthocyanidins (procyanidins and prodelphinidins) shows potential to inhibit pancreatic alpha-amylase. The degree of polymerization of proanthocyanidins has been demonstrated to be linearly related to alpha-amylase inhibitory activity (Lee et al., J Nutr Sci Vitaminol 2007, 53, 287-293; Horigome et al., Br J Nutr 1988, 60:275-85; Goncalves et al., Food Chemistry 2011, 125:665-672). Procyanidins of rowanberry and raspberry show a better alpha-amylase inhibition than anthocyanidins and ellagitannins (Grussu et al., J Agric Foo. Chem 2011, 59:2324-2331).

More specifically, Lu et al. claimed that procyanidins have hypoglycemic activities, irrespectively of the type of procyanidins. However, they did not propose a mechanism of action (Lu et al., Phytomedicin 2011, 18:298-302).

Pine bark extract (pinus pinaster) containing, among other molecules, oligomeric procyanidins has been reported to display antidiabetic effects (Liu et al., Diabetes Care, 2004, 27:839). It is believed that the antidiabetic effect of pine bark is due to its digestive enzyme inhibitory activity. (+)-catechin and procyanidins were shown to inhibit alpha-glucosidase and the activity of pine bark extract toward alpha-glucosidase increases with the degree of polymerization of procyanidins (Schafer and Hogger, Diabetes Res Clin Pract 2007, 77:41-6). In addition, pine bark extract has shown competitive inhibition against human salivary and porcine pancreas alpha-amylases (Kim et al., Nutrition 2005, 21:756-761).

Blade et al. in their review alleged that polymeric proanthocyanidins are not absorbable by the gut and can be locally active. They also stress the fact that polymeric proanthocyanidins positively influence the absorption of proanthocyanidins oligomers (Blade et al., Mol Nutr Food Res 2010, 54:37-59).

According to numerous authors the inhibitory activity of proanthocyanidins is due to the ability of those molecules to bind preferentially with protein to form a stabilized complex. Greater is the degree of polymerization greater is the number of molecule/protein interactions (Grussu et al., J Agric Foo. Chem 2011, 59:2324-2331; Blade et al., Mol Nutr Food Res 2010, 54:37-59).

The abundant prior art herein disclosed teaches various technical information in relation with the effects of various minerals and polyphenols on glucose metabolism. Some of the information disclosed may be contradictory and the most of the formulation described are not simply addressing the problem of postprandial hyperglycemia in a manner that is simple, safe and convenient.

The present inventors surprisingly and unexpectedly found that administration of a composition comprising or consisting in a combination of procyanidins and of a mineral mix comprising chromium, zinc, selenium, copper, magnesium and/or manganese, prior or simultaneously to the ingestion of a meal rich in carbohydrates, exhibits a synergistic effect and is able to acutely reduce postprandial blood glucose levels below the level of the additive reduction obtained with individual administration of procyanidins or of mineral components of the mix.

Indeed the synergistic effect of the composition according to the present invention is a great advantage since the quantity of procyanidins in the composition can be reduced by the complementation with the mineral mix. This is a benefit for the subject since the quantity of composition, in term of quantity of tablets to be swallowed for example, is much reduced compared to a traditional vegetal/herbal extract containing procyanidins alone.

The present invention thus concerns a composition comprising:
- procyanidins and
- a mineral mix comprising chromium and at least one mineral selected in the group consisting of zinc, selenium, copper, magnesium and manganese

The present invention thus concerns a composition comprising a combination of procyanidins and of a mineral mix comprising chromium and zinc and at least one mineral selected in the group consisting of, selenium, copper, magnesium and manganese.

The present invention thus concerns a composition comprising a combination of procyanidins and of a mineral mix comprising chromium, zinc and copper and at least one mineral selected in the group consisting of selenium, magnesium and manganese.

The present invention thus concerns a composition comprising a combination of procyanidins and of a mineral mix comprising chromium, zinc, copper and magnesium and at least one mineral selected in the group consisting of selenium and manganese.

The present invention thus concerns a composition comprising or consisting of a combination of procyanidins and of a mineral mix comprising or consisting of chromium, zinc, copper, magnesium and selenium.

The present invention thus concerns a composition comprising or consisting of a combination of procyanidins and of a mineral mix comprising or consisting of chromium, zinc, copper, magnesium and manganese.

The present invention thus concerns a composition comprising or consisting of a combination of procyanidins and of a mineral mix comprising or consisting of chromium, zinc, copper, selenium and manganese.

The present invention thus concerns a composition comprising or consisting of a combination of procyanidins and of a mineral mix comprising or consisting of chromium, zinc, copper, magnesium, selenium and manganese.

In one embodiment, the mineral mix comprises or consists in a mix of chromium and zinc.

In one embodiment, the mineral mix comprises or consists in a mix of chromium, zinc and copper.

In one embodiment, the mineral mix comprises or consists in a mix of chromium, zinc, copper and manganese.

In one embodiment, the mineral mix comprises or consists in a mix of chromium, zinc, copper and selenium.

In one embodiment, the mineral mix comprises or consists in a mix of chromium, zinc, copper, manganese and selenium. In one embodiment, the composition according to the invention is in the form of a tablet, a powder, a capsule.

In one embodiment, the present invention concerns a composition according to any of the previous embodiments, for use in the prevention or treatment, by oral administration prior to or simultaneously with the consumption of a food product rich in carbohydrates, of glucose related metabolism disorder.

In one embodiment, the oral administration is immediately after the consumption of a food rich in carbohydrates.

In one embodiment the disorder is selected in the group consisting of pre-diabetes, diabetes, hyperinsulinemia, hyperglycemia, metabolic syndrome, obesity and combinations thereof.

In one embodiment, the hyperglycemia is a postprandial hyperglycemia.

In one embodiment, the hyperglycemia is fasting hyperglycemia.

In one embodiment, the oral administration is one, two, three or four times a day, prior to or simultaneously with the meals.

In one embodiment the simultaneous oral administration comprises the incorporation of the composition in the food product prior to its consumption.

In one embodiment, the prevention or treatment of glucose related metabolism disorder consists in reduction of the Glycemic Index of the consumed food product by at least 20%, at least 25%, at least 30%, at least 35%, at least 40%.

The glycemic index (GI) is a classification of the blood glucose raising potential of carbohydrate foods. It is defined as the incremental area under the blood glucose response curve elicited by a 50 g digestible carbohydrate portion of a food expressed as a percentage of that after 50 g carbohydrate from a reference food taken by the same subject (wolever et al., Am J Clin Nutr 1991, 54:846-54).

In a further embodiment, the invention concerns a nutritional composition or a food product containing an effective amount of the composition according to the invention.

The effective amount of the composition according to the invention in the nutritional composition or the food product is comprised between 0.01 and 5% in weight of the nutritional composition or food product, particularly between 0,025 and 2.5%, more particularly between 0,05 and 1%, even more particularly between 0,1 and 0.5%.

In one embodiment, the nutritional composition or food product is rich in carbohydrates and contains, in % in weight, between 30 and 100% of carbohydrates, particularly between 40 and 100%, more particularly between 50 and 100%, more particularly between 60 and 100%, even more particularly between 70 and 100%, selected in the group of complex and simple carbohydrates and combination thereof.

The present disclosure provides methods of treating or preventing a disease or condition in a subject in need thereof comprising administering to the subject a therapeutically effective amount of a composition according to any embodiment of the invention.

The invention also concerns the composition of the invention for use as glycemic index lowering agent.

The invention also concerns the composition of the invention for use as weight management agent.

The invention also concerns the composition of the invention for use as satiety regulation agent.

In another embodiment, the therapeutically effective amounts of procyanidins and of mineral mix are present in the composition according to the invention in a synergistic amount.

In another embodiment, the composition according to any embodiment of the invention is administered to the subject weekly, daily, two times a day, or three times a day, prior to or simultaneously with the consumption of a food product rich in carbohydrates.

In another embodiment, the method is effective to achieve at least one of the following modifications: reduction in fasting or postprandial blood sugar level, reduction of HbA1c, decrease of insulin resistance, reduction of hyperinsulinemia, improvement of glucose tolerance, reduction of glycemic index, reduction of body weight or body weight gain, reduction of fat storage or any combination thereof.

In another embodiment, the therapeutically effective amount of the composition according to any embodiment of the invention is administered in conjunction with at least one additional treatment method, said additional treatment method comprising administering at least one pharmaceutical composition comprising an active agent selected from the group consisting of: a sulfonylurea, a meglitinide, a biguanide, an alpha-glucosidase inhibitor, a thiazolidinedione, a DPP-IV inhibitor, a glucagon-like peptide (GLP-1) analog, or insulin.

The present disclosure also provides a composition, nutritional composition, or medical food comprising a composition according to any embodiment of the invention for its use in the treatment or prevention of a disorder selected in the group consisting of pre-diabetes, diabetes, hyperinsulinemia, hyperglycemia, fasting hyperglycemia, postprandial hyperglycemia, metabolic syndrome, overweight, obesity, or any combination thereof.

In a preferred embodiment, the disorder is hyperglycemia. In another embodiment, the disorder is fasting hyperglycemia. In a further embodiment, the disorder is postprandial hyperglycemia.

In another embodiment, the composition is a pharmaceutical composition.

In another embodiment, the composition is a nutritional supplement composition.

In another embodiment, the composition is a nutritional composition.

In another embodiment, the composition is a food product.

In another embodiment, the invention concerns a kit comprising at least a procyanidin and a mix of minerals as described above for simultaneous, separate or sequential use in the prevention or treatment of a disorder selected in the group consisting of pre-diabetes, diabetes, hyperinsulinemia, hyperglycemia, fasting hyperglycemia, postprandial hyperglycemia, metabolic syndrome, overweight, obesity, or any combination thereof.

In a preferred embodiment, the disorder is hyperglycemia. In another embodiment, the disorder is fasting hyperglycemia. In a further embodiment, the disorder is postprandial hyperglycemia.

According to the invention, the oral administration of the composition according to the invention is done prior or simultaneously with the consumption of a food product rich in carbohydrates.

The expression prior to the consumption of a food product means that the composition is taken before or at the beginning of the meal.

In the context of the present invention, the expression "before the meal" means that the composition is taken in the few minutes, between 30 minutes and 0 minute, particularly between 15 minutes and 0 minute, more particularly about between 5 minutes and 0 minutes prior to the meal.

The expression "at the beginning of the meal" means that the composition is taken just before, or simultaneously to, the first bite of a meal.

In a further embodiment, the invention provides a method for regulating satiety in a subject comprising: administering to said subject an effective amount of a composition according to the invention, wherein said effective amount of composition is taken before or at the beginning of the meal.

In a further embodiment, the invention provides a method for preventing or reducing hyperglycemia in a subject in need thereof comprising: administering to said subject an effective amount of a composition according to the invention, wherein said effective amount of the composition is taken before or at the beginning of the meal.

The invention also provides a method for preventing obesity or diabetes in a subject in need thereof comprising: administering to said subject an effective amount of a composition according to the invention, wherein said effective amount of composition is taken before or at the beginning of the meal.

The invention also provides a method for preventing insulin resistance in a subject in need thereof comprising: administering to said subject an effective amount of a composition according to the invention, wherein said effective amount of composition is taken before or at the beginning of the meal.

The invention also provides a method for preventing metabolic syndrome in a subject in need thereof comprising: administering to said subject an effective amount of a composition according to the invention, wherein said effective amount of composition is taken before or at the beginning of the meal.

In a further embodiment, the invention relates to a method for reducing glycemic index of food, said method comprising adding to said food a composition according to the invention.

In one embodiment, the reduction of the glycemic index correspond to a reduction of 20 to 40%, particularly by about 20%, particularly about 25%, more particularly about 30%, more particularly about 35%, even more particularly about 40%.

Also is provided a method for reducing glycemic index of food rich in carbohydrates, said method comprising adding to said food a composition according to the invention, wherein the weight ratio composition/carbohydrate is of 5/100, 2.5/100, 1/100 or more, particularly 0.5/100, more particularly 0.2/100, more particularly about 0.1/100, 0.05/100, 0.01/100.

All dosage ranges contained within this application are intended to include all numbers, whole or fractions, contained within said range.

### DEFINITIONS.

As used herein, "subject" includes, but is not limited to, mammals, which include but is not limited to, rodents, aquatic mammals, domestic animals such as dogs and cats, farm animals such as sheep, pigs, cows and horses and humans. A subject may include a "patient" which is understood to include an animal, especially a mammal and more especially a human that is receiving or intended to receive treatment, as treatment is herein defined. While the terms "individual" and "patient" are often used herein to refer to a human, the present disclosure is not so limited. Accordingly, the terms "individual" and "patient" refer to any animal, mammal or human, having or at risk for a medical condition that can benefit from the treatment.

"Nutritional compositions", as used herein, are understood to include any number of optional additional ingredients, including conventional food additives, for example one or more, acidulants, additional thickeners, buffers or agents for pH adjustment, chelating agents, colorants, emulsifies, excipient, flavor agent, mineral, osmotic agents, a pharmaceutically acceptable carrier, preservatives, stabilizers, sugar, sweeteners, texturizers and/or vitamins. The optional ingredients can be added in any suitable amount.

The terms "prevention", "prevent", "preventing", "suppression", "suppress", "suppressing", "inhibit" and "inhibition" as used herein refer to a course of action (such as administering a compound or pharmaceutical composition) initiated in a manner (e.g., prior to the onset of a clinical symptom of a disease state or condition) so as to prevent, suppress or reduce, either temporarily or permanently, the onset of a clinical manifestation of the disease state or condition. Such preventing, suppressing or reducing need not be absolute to be useful.

The terms "treatment", "treat" and "treating" as used herein refers a course of action (such as administering a compound or pharmaceutical composition) initiated after the onset of a clinical symptom of a disease state or condition so as to eliminate, reduce, suppress or ameliorate, either temporarily or permanently, a clinical manifestation or progression of the disease state or condition. Such treating need not be absolute to be useful.

As used herein, the term "in need of treatment" refers to a judgment made by a caregiver that a patient requires or will benefit from treatment. This judgment is made based on a variety of factors that are in the realm of a caregiver's expertise, but that includes the knowledge that the patient is ill, or will be ill, as the result of a condition that is treatable by a method or compound of the disclosure.

As used herein, the term "in need of prevention" refers to a judgment made by a caregiver that a patient requires or will benefit from prevention. This judgment is made based on a variety of factors that are in the realm of a caregiver's expertise, but that includes the knowledge that the patient will be ill or may become ill, as the result of a condition that is preventable by a method or compound of the disclosure.

As used herein, the term "therapeutically effective amount" refers to an amount of the composition according to the invention, either alone or as a part of an other pharmaceutical, nutritional composition or food product, that is capable of having any detectable, positive effect on any symptom, aspect, or characteristics of a disease state or condition when administered to a patient (e.g., as one or more doses). Such effect need not be absolute to be beneficial.

The term "insulin resistance" as used herein refers to a condition where a normal amount of insulin is unable to produce a normal physiological or molecular response. In some cases, a hyper-physiological amount of insulin, either endogenously produced or exogenously added, is able to overcome the insulin resistance in whole or in part and produce a biologic response.

The term "carbohydrate" as used herein refers to a biological molecule consisting of carbon (C), hydrogen (H) and oxygen (O) atoms, usually with a hydrogen-oxygen atom ratio of 2:1 (as in water); in other words, with the empirical formula C*ₘ*(H₂O)*ₙ* (where m could be different from n). The term is most common in biochemistry, where it is a synonym of saccharide, a group that includes sugars, starch, and cellulose. The saccharides are divided into four chemical groups: monosaccharides, disaccharides, oligosaccharides, and polysaccharides. In general, the monosaccharides and disaccharides, which are smaller (lower molecular weight) carbohydrates, are commonly referred to as sugars In food science and in many informal contexts, the term carbohydrate often means any food that is particularly rich in the complex carbohydrate starch (such as cereals, bread and pasta) or simple carbohydrates, such as sugar (found in candy, jams, and desserts). Carbohydrates are polyhydroxy aldehydes, ketones, alcohols, acids, their simple derivatives and their polymers having linkages of the acetal type. They may be classified according to their degree of polymerization and may be divided initially into three principal groups, namely sugars, oligosaccharides and polysaccharides.

In the context of the present invention, the carbohydrates are a digestible carbohydrates which excludes the non-digestible ones such as cellulose, hemicellulose and pectins, for example.

Accordingly the carbohydrates comprise complex carbohydrates and simple carbohydrates.

The complex carbohydrates comprise oligosaccharides and polysaccharides.

The oligosaccharides comprise malto-oligosaccharides and other oligosaccharides such as maltodextrins, raffinose, stachyose and fructo-oligosaccharides.

The polysaccharides comprise starch and non-starch polysaccharides. Starch comprises amylose and amylopectine as well as modified starches.

Simple carbohydrates comprise sugars. Sugars comprise monosaccharides, disaccharides. Monosaccharides comprise glucose, fructose, xylose, galactose, for example.

Disaccharides comprise sucrose, lactose, maltose, trehalose, for example.

Methods of treating or preventing a disease or condition (e.g., hyperinsulinemia, hyperglycemia, fasting or postprandial hyperglycemia, metabolic syndrome, type 2 diabetes, type 1 diabetes and obesity) in a subject in need thereof, are provided by the disclosure and comprise administering to the subject a therapeutically effective amount of a composition according to any of the embodiment herein described.

The inventors have unexpectedly found that a composition according to the invention as herein described when administered to a subject reduces postprandial blood glucose levels below that achieved with administration of either procyanidins or minerals or mineral mix alone (i.e., exhibits a synergistic effect).

In some embodiments, the method is sufficient to achieve at least one of the following modifications: reduction in fasting or postprandial blood sugar level, decrease of insulin resistance, reduction of hyperinsulinemia and/or improvement of glucose tolerance, reduction of glycemic index, reduction of body weight or body weight gain, reduction of fat storage or any combination thereof.

In some embodiments, the treatment or prevention method reduces a symptom of diabetes or the chances of developing a symptom of diabetes, wherein the symptom is selected from the group consisting of: atherosclerosis, obesity, hypertension, hyperlipidemia, fatty liver disease, nephropathy, neuropathy, retinopathy, foot ulceration and cataracts, associated with diabetes.

Optionally, administration of the therapeutically effective amount of a composition according to any embodiments herein described results in an improvement in glycemic control in the subject.

The subject may be a healthy subject or a subject already exhibiting a disorder selected in the group consisting of hyperinsulinemia, hyperglycemia, fasting or postprandial hyperglycemia, metabolic syndrome, type 2 diabetes, type 1 diabetes, overweight, obesity.

Compositions according to the invention may comprise, beside the therapeutically or prophylactically effective amount (e.g., a synergistic amount) of procyanidins and a mineral mix, a pharmaceutically acceptable agent.

Pharmaceutically acceptable agents include carriers, excipients, diluents, antioxidants, preservatives, coloring, flavoring and diluting agents, emulsifying agents, suspending agents, solvents, fillers, bulking agents, buffers, delivery vehicles, tonicity agents, co-solvents, wetting agents, complexing agents, buffering agents, antimicrobials and surfactants.

Suitable and/or preferred pharmaceutical formulations can be determined in view of the present disclosure and general knowledge of formulation technology, depending upon the intended route of administration, delivery format and desired dosage. Regardless of the manner of administration, an effective dose can be calculated according to patient body weight, body surface area, or organ size. Further refinement of the calculations for determining the appropriate dosage for treatment involving each of the formulations described herein are routinely made in the art and is within the ambit of tasks routinely performed in the art. Appropriate dosages can be ascertained through use of appropriate dose-response data.

A therapeutically effective amount refers to an amount effective, at dosages and for periods of time necessary, to achieve the desired therapeutic result. A therapeutically effective amount may vary according to factors such as the disease state, age, sex and weight of the individual and the ability to elicit a desired response in the individual. A therapeutically effective amount is also one in which any toxic or detrimental effects are outweighed by the therapeutically beneficial effects. A prophylactically effective amount refers to an amount effective, at dosages and for periods of time necessary, to achieve the desired prophylactic result.

The composition may be in the form of tablets, capsules, pastilles or a liquid for example. The supplement may further contain protective hydrocolloids (such as gums, proteins, modified starches), binders, film forming agents, encapsulating agents/materials, wall/shell materials, matrix compounds, coatings, emulsifiers, surface active agents, solubilizing agents (oils, fats, waxes, lecithins or the like), adsorbents, carriers, fillers, co-compounds, dispersing agents, wetting agents, processing aids (solvents), flowing agents, taste masking agents, weighting agents, jellifying agents and gel forming agents. The supplement may also contain conventional pharmaceutical additives and adjuvants, excipients and diluents, including, but not limited to, water, gelatin of any origin, vegetable gums, ligninsulfonate, talc, sugars, starch, gum arabic, vegetable oils, polyalkylene glycols, flavoring agents, preservatives, stabilizers, emulsifying agents, buffers, lubricants, colorants, wetting agents, fillers and the like.

The composition can be added in a product acceptable to the consumer (who is a human or an animal), such as an ingestible carrier or support, respectively. Examples of such carriers or supports are a pharmaceutical or a food or a pet food composition. Non-limiting examples for such compositions are milk, yogurt, curd, cheese, fermented milks, milk based fermented products, fermented cereal based products, milk based powders, human milk, preterm formula, infant formula, oral supplements and tube feeding.

The composition can be a dry powdered formulation. The powdered formulation can be made by combining dry powdered ingredients or can be made from a liquid nutritional composition by drying the liquid composition with one of the processes known in the art, including spray drying, freeze drying or other drying techniques, to produce a dry powdered composition. If desired, other nutritional components or compositions can be added to the liquid prior to drying to provide enhanced nutritional benefits to the powdered formulation. Such powdered formulations have a much greater shelf life and can be packaged for storage and transport for future use. At that time, the powdered formulations can be reconstituted with water or other fluids and then administered to the individual orally or enterally.

Alternatively, the powdered composition can be dry-mixed with the food or meal to be consumed extemporaneously prior to its consumption.

The powdered formulation can be packaged in various containers, including those for bulk provision of such powdered formulations for adding to a liquid in a glass, bottle or other fluid containing vessel, or a single serving can be provided with the powder present in a container to which water or other fluids can be added to form the liquid for oral or enteral administration.

The present inventors surprisingly and unexpectedly identified a synergy of procyanidins and minerals on the glucose metabolism.

To the best knowledge of the inventors, this is the first time that the synergy of this combination has been shown.

The quantity of procyanidins and minerals here below indicated are given for a single oral dosage format of the composition. The dose regimen to be administered orally to a subject may vary depending of the age, the weight and the pathology of such a subject and of the procynidins and minerals concentration of the said single oral dosage format.

As a matter of consequence the number of single oral dosage format of a composition according to the invention to be administered orally can vary, on a daily basis and/or on a meal basis, from 1 to 20 single oral dosage format, particularly from 1 to 10, more particularly from 1 to 5, even more particularly from 1 to 3.

For the sake of convenience, the number of single oral dosage format will be as limited as possible and particularly the composition according to the invention is to be administered as 1 or 2 single oral dosage format per meal, i.e. immediately prior, simultaneously, or immediately after the consumption of a meal.

The quantity of procyanidins in the composition of the invention may vary, for a single oral dosage format of the composition, from 5 to 500 mg, particularly from 20 to 200 mg, particularly from 40 to 100 mg, even more particularly about 70 mg.

The procyanidins may be selected in the group consisting of epicatechin, epigallocatechin, epicatechine gallate, catechin, gallocatechin, gallocatechin gallate and their mixtures.

In an embodiment, the procyanidins of the composition according to the invention comprise or consist in pure procyanidins purified from a plant origin.

In one embodiment, the procyanidins of the composition according to the invention comprises or consist in a plant extract rich in procyanidins.

Rich plant sources of procyanidins are grapes, particularly grapes seed and grapes skin, cocoa, apples, peanuts, almonds, hazelnuts, pecans, pistachios, walnuts, cranberries, blueberries, black berries, choke berries, bilberry, black currant, strawberries, raspberries, plums, bark of the maritime pine, cinnamon, aronia, acai, green tea, black tea, sorghum, red beans, pinto beans...

Accordingly, the procyanidins of the composition of the invention may be in the form of a plant extract rich in procyanidins selected in the group consisting of, but not limited to, grapes, cocoa, apples, peanuts, almonds, hazelnuts, pecans, pistachios, walnuts, cranberries, blueberries, black berries, choke berries, bilberry, black currant, strawberries, raspberries, plums, bark of the maritime pine, cinnamon, aronia, acai, green tea, black tea, sorghum, red beans, pinto beans.

The plant extract rich in procyanidins may contain from 5 to 100%, by weight of procyanidins, particularly from 20 to 60 %, more particularly from 30 to 50%, more particularly about 40%.

In one particular embodiment, the plant extract rich in procyanidins used in the composition of the invention is a cinnamon extract that provides at least a portion or the totality of the procyanidins of the composition.

The cinnamon extract can be an extract from the bark of Cinnamomum verum also called Cinnamomum zeylanicum or an extract from the bark of Cinnamomum aromaticum also called Cinnamomun cassia or a mixture thereof.

In one preferred embodiment, the cinnamon extract is a hydro-alcoholic extract of Ceylon cinnamon bark also called Cinnamomum verum and Cinnamomum zeylanicum.

In an embodiment, the cinnamon extract is an extract of Cinnamomum verum, susceptible of being obtained by the process comprising the following steps:
a) contacting dried milled Cinnamomum verum bark with an extraction alcoholic or hydro-alcoholic solvent at a temperature of around 50°C,
b) filtering out the solids and collecting alcoholic extract
c) washing said solids by stirring with an alcoholic or hydro-alcoholic extraction solvent
d) filtering out the solids and collecting alcoholic or hydro-alcoholic extract
e) combining said alcoholic extracts
f) removing non-soluble residues by decantation or filtration
g) evaporating the solvents from the combination at a temperature inferior or equal to 50°C
h) recovering a dry extract.

In a preferred embodiment the cinnamon extract is an alcoholic extract as described in EP2654460.

The cinnamon extract may come from the bark of the cinnamon, in particular of Cinnamomum verum, in an amount of at least 75 % by weight, in particular at least 90 % by weight, more particularly at least 95 % by weight compared to the total weight of the cinnamon extract.

Following an embodiment, 100 % of the cinnamon, in particular the Cinnamomum verum, extract is coming from the bark.

The preferred cinnamon extract is standardized at a minimum of 40% of polyphenols. These polyphenols mainly comprise procyanidin oligomers comprising catechin and epicatechin monomers.

Proanthocyanidins are polymers or oligomers of flavan-3-ols (see Table 1). Proanthocyanidins can have a variety of structure due to: different chain length, different number of hydroxyl groups, different stereochemistry, different location and type of interflavan linkage. Doubly linked flavan-3-ols are called A-type and simply linked are called B-type.

**Table 1 Most occurring flavan-3-ols in nature**

| Most important flavan-3-ols naturally occuring | | | |
|---|---|---|---|
| | R3 | R5' | Structures |
| (-)-epicatechin EC | OH | H | 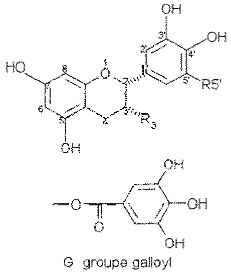 |
| (-)-epigallocatechine EGC | OH | OH | |
| (-)-epicatechine gallate ECG | G | H | |
| (-)-epigallocatechine gallate EGCG | G | OH | |
| (+)-catechin C | OH | H | 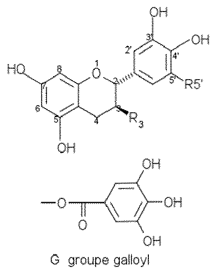 |
| (+)-gallocatechin GC | OH | OH | |
| (+)-gallocatechin gallate GCG | G | OH | |

More specifically, proanthocyanidins based on catechin and epicatechin building blocks are the most encountered in nature and are called procyanidins. Other important proanthocyanidins groups are based on gallocatechin, epigallocatechin and epigallocatechin gallate and are called prodelphinidins (Blade et al., Mol Nutr Food Res 2010, 54:37-59).

In an embodiment, the procyanidins of the composition according to the invention are selected in the group consisting of epicatechin, epigallocatechin, epicatechine gallate, catechin, gallocatechin, gallocatechin gallate and their mixtures.

In one embodiment, the quantity of procyanidins in a single oral dosage format of the composition of the invention may range from 5 to 500 mg, particularly from 20 to 200 mg, particularly from 40 to 100 mg, even more particularly about 70 mg.

The minerals of the composition according to the present invention may be of any origin, either organic or mineral. By organic origin, it is meant that the mineral mix may come from a bacterial, animal, plant, algae or yeast extract. Organic may also mean that the mineral element is combined in the form of a salt with an organic acid, for example.

Preferred forms of zinc include zinc salts such as zinc acetate, zinc L-ascorbate, zinc L-aspartate, zinc bisglycinate, zinc chloride, zinc citrate, zinc gluconate, zinc lactate, zinc L-lysinate, zinc malate, zinc mono-L-methionine sulphate, zinc oxide, zinc carbonate, zinc L-pidolate, zinc picolinate, for example.

Preferred forms of chromium include chromium chloride, chromium lactate trihydrate chromium nitrate, chromium picolinate, chromium sulphate, for example.

Preferred forms of Magnesium include magnesium acetate, magnesium L-ascorbate, magnesium bisglycinate, magnesium carbonate, magnesium chloride, magnesium salts of citric acid, magnesium gluconate, magnesium glycerophosphate, magnesium salts of orthophosphoric acid, magnesium lactate, magnesium L-lysinate, magnesium hydroxide, magnesium malate, magnesium oxide, magnesium L-pidolate, magnesium potassium citrate, magnesium pyruvate, magnesium succinate, magnesium sulphate, magnesium taurate, magnesium acetyl taurate, for example.

Preferred forms of manganese include manganese ascorbate, manganese L-aspartate, manganese bisglycinate, manganese carbonate, manganese chloride, manganese citrate, manganese gluconate, manganese glycerophosphate, manganese pidolate, manganese sulphate, for example.

Preferred forms of copper include cupric carbonate, cupric citrate, cupric gluconate, cupric sulphate, copper L-aspartate, copper bisglycinate, copper lysine complex, copper oxide, for example.

Preferred forms of selenium include L-selenomethionine, selenium enriched yeast, selenious acid, sodium selenite, sodium hydrogen selenite, sodium selenite, for example.

In one embodiment, the mineral mix comprises or consists in extracts of bacteria, plants, algae or yeast enriched in minerals.

As exposed above, the quantity of active ingredients in the composition according to the invention is expressed in weight for a single oral dosage format.

Similarly the composition according to the invention can comprise mineral or minerals in an amount ranging, for a one dosage format, from few µg to hundreds of mg.

However, as a guidance and in a preferred embodiment, a composition according to the invention may comprise, in a single oral dosage format:
- Procyanidins ; particularly in the form of a dried hydro-alcoholic extract of Ceylon cinnamon bark containing in % in weight at least 20% of procyanidins, in particularly between 20 and 100% of procyanidins, more particularly between 30 and 60% of procyanidins, more particularly around 45% of procyanidins ; in a quantity ranging from 5 to 500 mg, particularly from 20 to 200 mg, particularly from 40 to 100 mg, even more particularly between about 60 and 80 mg;
- Zinc in a quantity comprised between 1 and 20 mg, particularly between 2 and 10 mg, more particularly between about 3 and 4 mg;
- Selenium in a quantity comprised between 0.001 and 0.2 mg, particularly between 0.005 and 0.05 mg, more particularly between about 0.01 and 0.015 mg;
- Manganese in a quantity comprised between 0.1 and 10 mg, particularly between 0.5 and 2 mg, more particularly between about 0.8 and 0.9 mg;
- Copper in a quantity comprised between 0.05 and 10 mg, particularly between 0.1 and 1 mg, more particularly between about 0.4 and 0.6 mg;
- Chromium in a quantity comprised between 0.5 and 50 µg, particularly between 1 and 15 µg, more particularly between about 5 and 7 µg.

The quantity of each of the minerals expressed here above does correspond to the quantity of the mineral element and not to the chemical form or chemical origin, whether it is a salt or any mineral form of the element.

In a further embodiment, the composition according to the invention is for its use in the treatment or prevention of conditions linked to foods with a high or medium glycemic index.

In another embodiment, the invention concerns a composition according to the present invention wherein the conditions linked to foods with a high glycemic index are selected from the group consisting of impaired glucose tolerance (IGT), impaired fasting glucose (IFG), hyperinsulinemia, diabetes, overweight, obesity, metabolic syndrome, high blood pressure, high blood cholesterol and heart diseases.

In another embodiment, the invention concerns composition in accordance with the invention for its use for at least partially reducing glucose peaks and/or insulin peaks in the blood of a consumer after consumption of a meal or beverage rich in digestible carbohydrates.

In another embodiment, the invention relates to composition according to the invention for its consumption immediately before, during and/or immediately after a meal or beverage.

Hence, the present invention allows using the generally well liked easily digestible carbohydrates in food products while having the advantage that these carbohydrates are in fact slowly digested. Hence, a similar effect is achieved as if one was using more complex carbohydrates, while taste issues are avoided.

The glycemic index (GI) is a measure of the effects of carbohydrates on blood sugar levels. Carbohydrates that break down quickly during digestion and release glucose rapidly into the bloodstream have a high GI; carbohydrates that break down more slowly, releasing glucose more gradually into the bloodstream, have a medium or even a low GI. The glycemic index of a food is defined as the area under the two hour blood glucose response curve (AUC) following the ingestion of a fixed portion of carbohydrate (usually 50 g). The AUC of the test food is divided by the AUC of the standard (glucose) and multiplied by 100. Glucose has hence a GI of 100 per definition.

Food compositions with a GI of superior or equal to 70 shall be understood as food composition with a high glycemic index. Food compositions with a GI from 69 to 56 shall be understood as food composition with a medium glycemic index. Food compositions with a GI of inferior or equal to 55 shall be understood as food composition with a low glycemic index.

The meal, expression which also include beverage, may be a food rich in digestible carbohydrates and may also be rich in sucrose and/or maltose. It allows increasing the time span during which a body can use ingested food as energy source. Hence, the composition of the present invention may be used to increase the time span during which energy can be extracted from ingested food.

It also allows prolonging the feeling of satiety after consumption of the composition.

The disorders linked to foods with a high glycemic index that may be treated or prevented by the composition of the present invention may be selected from the group consisting of impaired glucose tolerance (IGT), impaired fasting glucose (IFG), hyperinsulinemia, diabetes, overweightness, obesity, high blood pressure, high blood cholesterol and heart diseases.

Immediately before or immediately after, in respect of the oral administration of the composition according to the invention in conjunction with the consumption of a meal shall be understood as the time frame ranging from about 30 minutes before or after the ingestion of meal, preferably about 15 minutes before or after the ingestion of a meal.

### EXAMPLES

### Example 1: Production and analysis of cinnamon procyanidin extract (CPE)

The cinnamon procyanidin extract (named CPE) tested in this study is a hydro-alcoholic extract of Ceylon cinnamon bark (10:1), also called true Cinnamon, *Cinnamomum zeylanicum,* or *Cinnamomum verum.* The dried and milled cinnamon barks were extracted with a 50:50 (v/v) water-ethanol solution during a 2 hour period. After a first solid-liquid separation, the remaining cinnamon barks were extracted a second time with a 50:50 (v/v) water-ethanol solution. The 2 eluates were combined together. The extraction mixture was filtrated in order to remove any remaining solids, then concentrated under reduced pressure through evaporation of the ethanol and most of the water, before finally being dried in a vacuum drier to obtain a fine brown powder.

CPE is standardized at a minimum of 40% of polyphenols. These polyphenols are mainly constituted of procyanidin oligomers which are composed of catechin and epicatechin monomers.

The total polyphenol content was measured by a colorimetric method using a Folin-Ciocalteu reagent as per the ISO14502-1 method. The total procyanidins content was measured by a colorimetric method using the DMAC reagent according to BL-DMAC standardized operation procedure published by International Chemistry Testing.

According to these methods, the CPE tested in the following examples was titrated at 48.7% of dry extract of polyphenols and at 43.9 % of dry extract of procyanidins.

### Example 2: Minerals and mix of minerals

Various minerals and various forms of minerals were used for these studies. In some examples below, chemical minerals or mix of minerals were tested, notably chromium picolinate, chromium nicotinate (also called polynicotinate), zinc acetate dihydrate, selenomethionine, copper acetate, magnesium oxide, manganese citrate, manganese chloride. In Example 11 below, yeast (baker's yeast) enriched in minerals, notably enriched in zinc, chromium and selenium, and mix thereof were tested.

### Example 3: Dose-effect of cinnamon procyanidin extract (CPE on the glycemic response to starch in rat

Five-week old male Wistar rats weighing between 50 and 75 g were purchased from Janvier Labs. Two rats were housed per cage. Filtered tap water and regular animal non-purified diet comprised of 51.7% carbohydrates, 21.4% proteins, and 5.1 % lipids (diet A03, SAFE) were supplied *ad libitum*. The animal room environment was controlled with a temperature of 22 ± 2 °C and day/night cycles of 12 hours light, 12 hours dark (19:00-7:00). Animals were allowed to acclimate to the laboratory environment for 10 days, weighed 3 times per week, and randomly assigned to the different study groups (8 to 16 animals per group depending on the study, as indicated in each example). The protocols were approved by the following ethics committee: *"*Comité Regional d'Ethique sur l'Expérimentation Animale", Pharmacology Laboratory, Pharmacy University, 15 Avenue Charles Flahault, 34093 Montpellier, France. Starch tolerance tests (STT) were conducted in animals which had fasted overnight through the acute administration by oral gavage of a 7.5% wheat starch solution at 1.5 g/kg or 20 mL/kg of body weight containing the products to be tested. The control group was administered starch only. The actual volume administered to each rat was calculated and adjusted based on the most recent body weight of each animal. Blood samples were collected via the tail vein before and 15, 30, 45, 60, 90, and 120 min after starch administration. One drop of blood was used for the glucose determination using a hand-held glucometer (OneTouch Ultra 2, LifeScan).

In this Example 3, the effect of starch alone and starch containing escalating doses of cinnamon procyanidin extract (named CPE): 6.25, 12.5, 25, 50, and 100 mg/kg of body weight were compared.

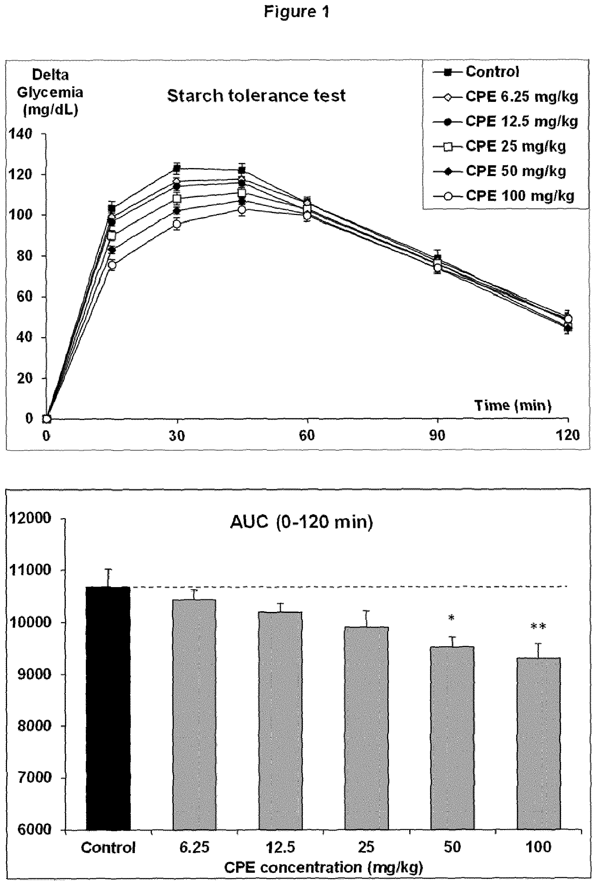

The figure 1 represents mean values +/- standard error of the mean (sem; n=8) and shows that the CPE reduced the glycemic response to starch in a dose-dependent manner. On the graph representing the area under the curve (AUC) between 0 and 120 min, this effect is statistically significant from 50 mg/kg of body weight (t-test p<0.05).

### Example 4: Dose-effect of chromium nicotinate on the glycemic response to starch in rat

The protocol was the same as for Example 3.

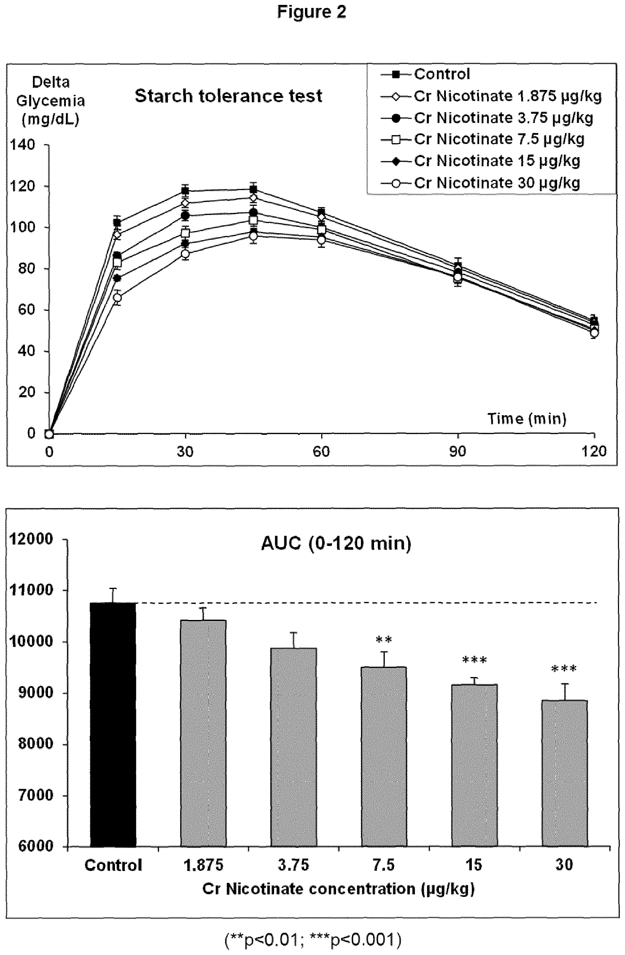

The figure 2 represents mean values +/- standard error of the mean (sem; n=8) and shows that the chromium nicotinate reduced the glycemic response to starch in a dose-dependent manner. On the graph representing the area under the curve (AUC) between 0 and 120 min, this effect is statistically significant from 7.5 µg/kg of body weight (t-test p<0.01).

### Example 5: Combination of cinnamon procyanidin extract (CPE) and chromium nicotinate on the glycemic response to starch in rat

Synergism is define by the ability of two or several combined active principles to display an effect that is greater than what can be predicted by their individual potencies. The potency of an active principle depends on its dose and is defined by its dose-effect curve which may greatly vary from an active principle to another.

The isobole method was used for assessing product synergism. Practically, the demonstration of synergism uses the concept of dose equivalence in order to make an isobole analysis. The isobologram uses a straight line which represents every combination of doses that are additive. After the construction of the isobologram, different combinations (with different concentrations) are tested, plotted on the isobologram and compared to the line representing the additive effect. If the point is plotted below the isobole, the effect is superior to the expected additive effect and the combination is synergistic. If it is above the isobole, the effect is inferior to the expected additive effect and the combination is antagonist. Finally, if the point is on the isobole, the effect is additive (Chou T.C., Drug combination studies and their synergy quantification using the Chou-Talalay method. Cancer research, 2010. 70(2): p. 440-446 and Tallarida R.J., Quantitative methods for assessing drug synergism. Genes & cancer, 2011. 2(11): p. 1003-1008).

In the present studies, isoboles and combination indexes were calculated thanks to the free software named CompuSyn (Chou T. and Martin N., CompuSyn for drug combinations, PC software and user's guide: a computer program for quantitation of synergism and antagonism in drug combinations, and the determination of IC50 and ED50 and LD50 values. ComboSyn, Paramus, NJ, 2005). Results are given in the form of a graphic, the isobologram, and in the form of a number named combination index (CI). If Cl = 1, the combined products are additive, if Cl < 1 they are synergistic, and if Cl > 1, they are antagonist.

The protocol was the same as for Example 3.

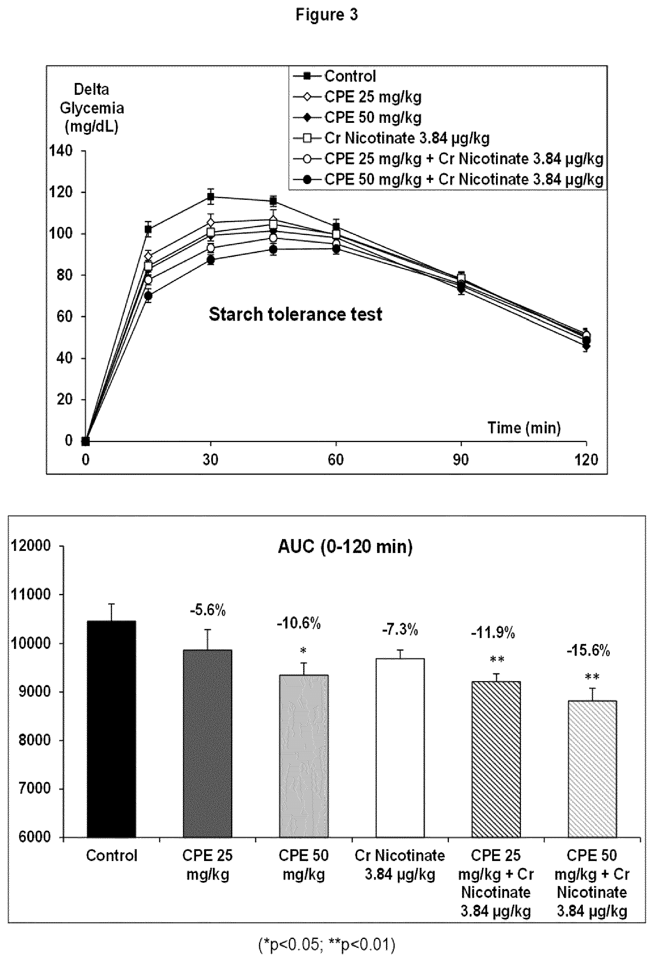

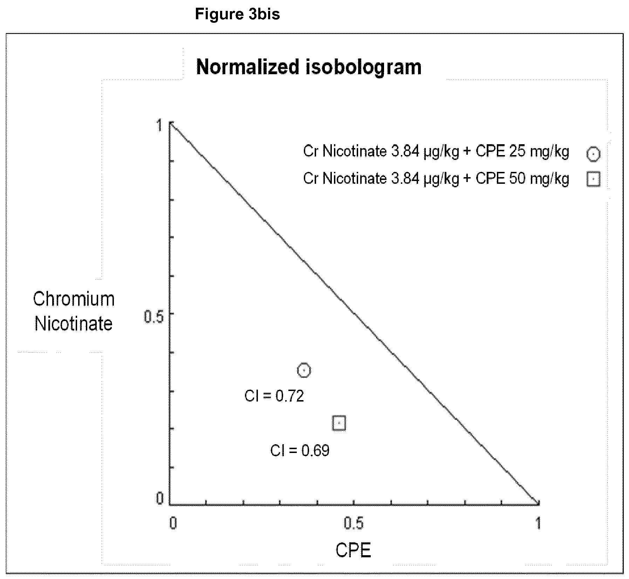

The figure 3 represents mean values +/- standard error of the mean (sem; n=8) and shows that the CPE reduced the glycemic response to starch by 5.6% at 25 mg/kg of body weight and by 10.6% at 50 mg/kg of body weight (t-test p<0.05), whereas the chromium nicotinate at 3.84 µg/kg of body weight reduced the glycemic response to starch by 7.3%. When the 2 products were given together, the effect reached 11.9% (t-test p<0.01) and 15.6% (t-test p<0.01) for the combination with 25 and 50 mg/kg of body weight of CPE, respectively.

The isobole (shown on Figure 3 bis )analysis revealed that CPE and chromium nicotinate have a synergistic effect on the glycemic response (Cl=0.72 and 0.69 for the combination with 25 and 50 mg/kg of body weight of CPE, respectively).

### Example 6: Dose-effect of chromium picolinate on the glycemic response to starch in rat

The protocol was the same as for Example 3.

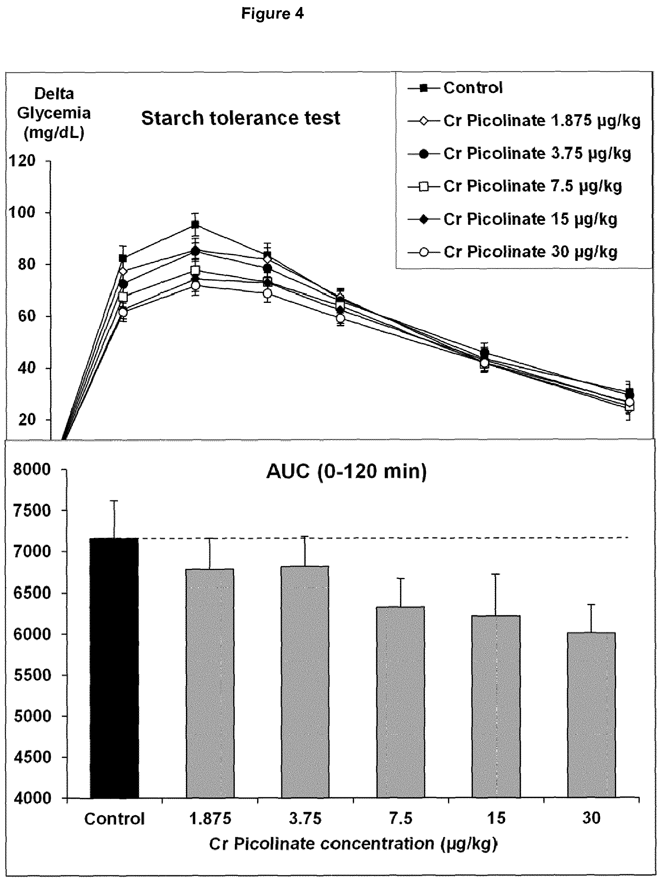

The figure 4 represents mean values +/- standard error of the mean (sem; n=8) and shows that the chromium picolinate reduced the glycemic response to starch in a dose-dependent manner. On the graph representing the area under the curve (AUC) between 0 and 120 min, this effect is nearly statistically significant at 30 µg/kg of body weight (t-test p=0.063).

### Example 7: Combination of cinnamon procyanidin extract (CPE) and chromium picolinate on the glycemic response to starch in rat

The protocol was the same as for Examples 3 and 5.

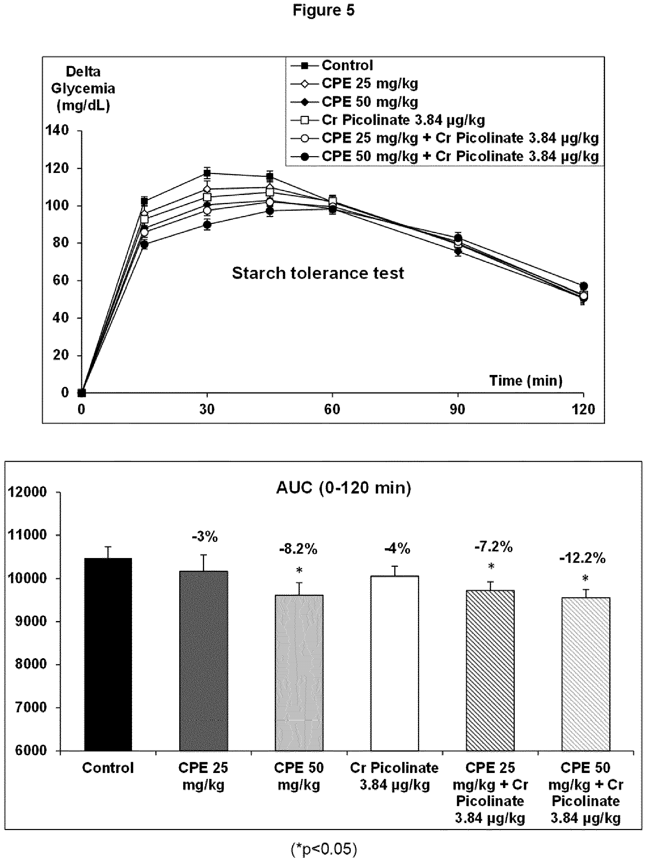

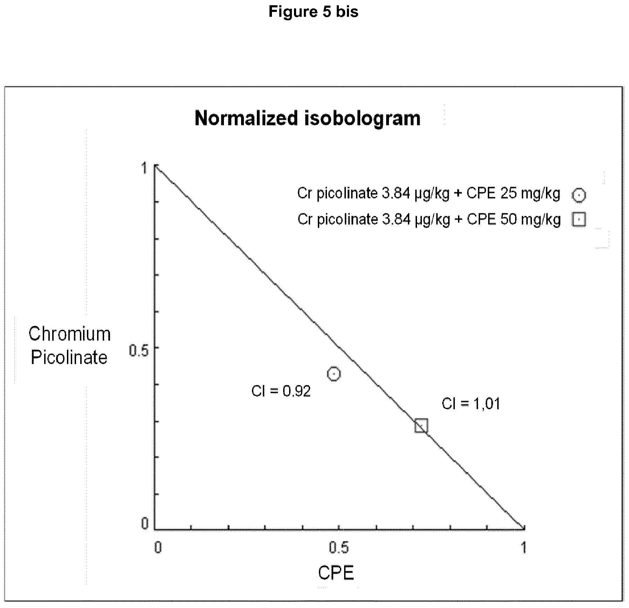

The figure 5 represents mean values +/- standard error of the mean (sem; n=8) and shows that the CPE reduced the glycemic response to starch by 3% at 25 mg/kg of body weight and by 8.2% at 50 mg/kg of body weight (t-test p<0.05), whereas the chromium picolinate at 3.84 µg/kg of body weight reduced the glycemic response to starch by 4%. When the 2 products were given together, the effect reached 7.2% (t-test p<0.05) and 12.2% (t-test p<0.05) for the combination with 25 and 50 mg/kg of body weight of CPE, respectively.

The isobole (shown on figure 5 bis) analysis revealed that CPE and chromium picolinate have an additive or nearly additive effect on the glycemic response (Cl=0.92 and 1.01 for the combination with 25 and 50 mg/kg of body weight of CPE, respectively).

### Example 8: Combination of cinnamon procyanidins extract (CPE and magnesium oxide, copper acetate, zinc acetate, manganese chloride on the glycemic response to starch in rat

The protocol was the same as for Example 3.

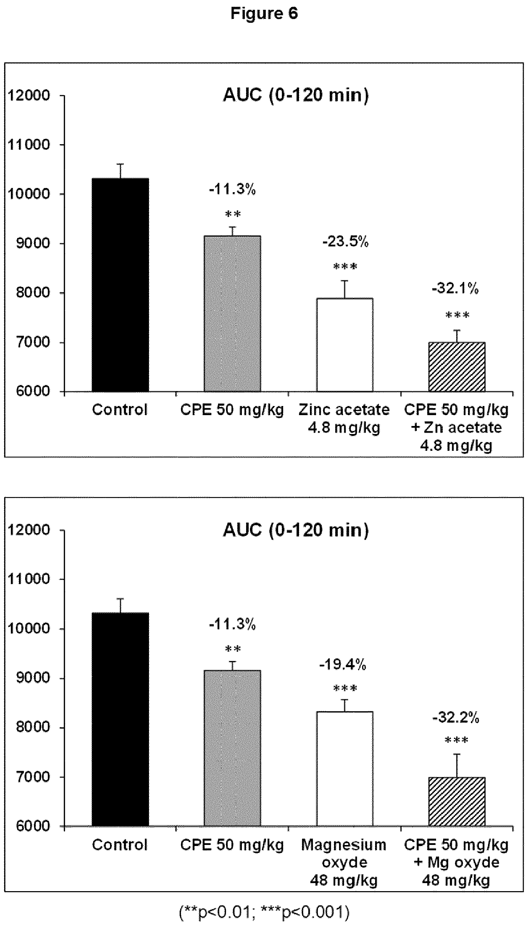

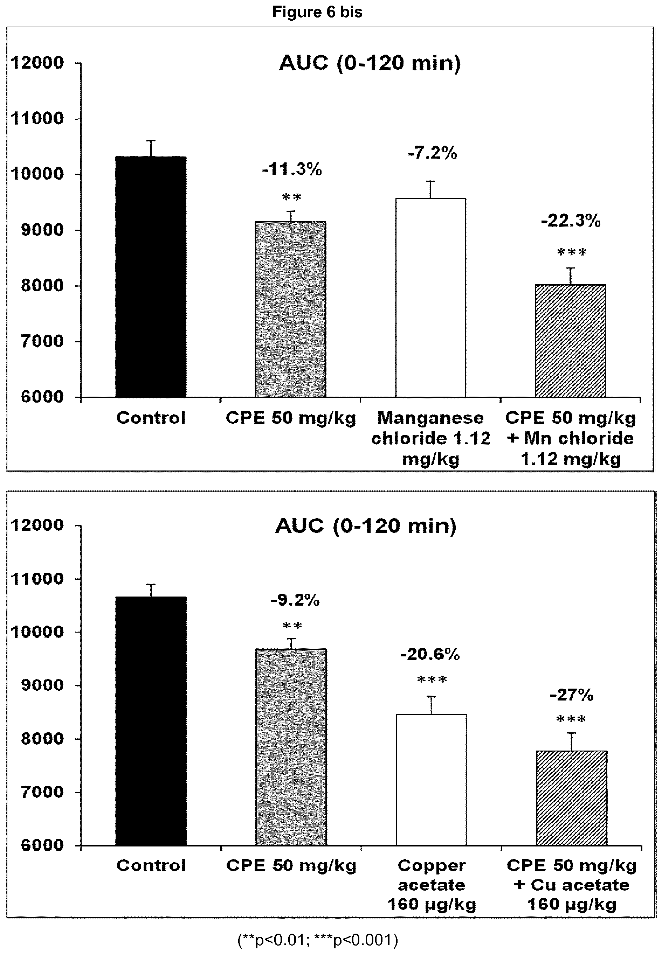

The figure 6 represents mean values +/- standard error of the mean (sem; n=8 for all the conditions but the Control and the CPE 50 mg/kg conditions where n=16) and shows combinations of the CPE with different minerals.

The first panel shows that the CPE at 50 mg/kg of body weight reduced the glycemic response to starch by 11.3% (t-test p<0.01) whereas zinc acetate at 4.8 mg/kg of body weight reduced the glycemic response to starch by 23.5% (t-test p<0.001). When the 2 products were given together, the effect reached 32.1% (t-test p<0.001).

The second panel shows that the CPE at 50 mg/kg of body weight reduced the glycemic response to starch by 11.3% (t-test p<0.01) whereas magnesium oxide at 48 mg/kg of body weight reduced the glycemic response to starch by 19.4% (t-test p<0.001). When the 2 products were given together, the effect reached 32.2% (t-test p<0.001).

The third panel shows that the CPE at 50 mg/kg of body weight reduced the glycemic response to starch by 11.3% (t-test p<0.01) whereas manganese chloride at 1.12 mg/kg of body weight reduced the glycemic response to starch by 7.2%. When the 2 products were given together, the effect reached 22.3% (t-test p<0.001).

The fourth panel shows that the CPE at 50 mg/kg of body weight reduced the glycemic response to starch by 9.2% (t-test p<0.01) whereas copper acetate at 0.16 mg/kg of body weight reduced the glycemic response to starch by 20.6% (t-test p<0.001). When the 2 products were given together, the effect reached 27% (t-test p<0.001).

### Example 9: Dose-effect of a mix of minerals on the glycemic response to starch in rat

The protocol was the same as for Example 3.

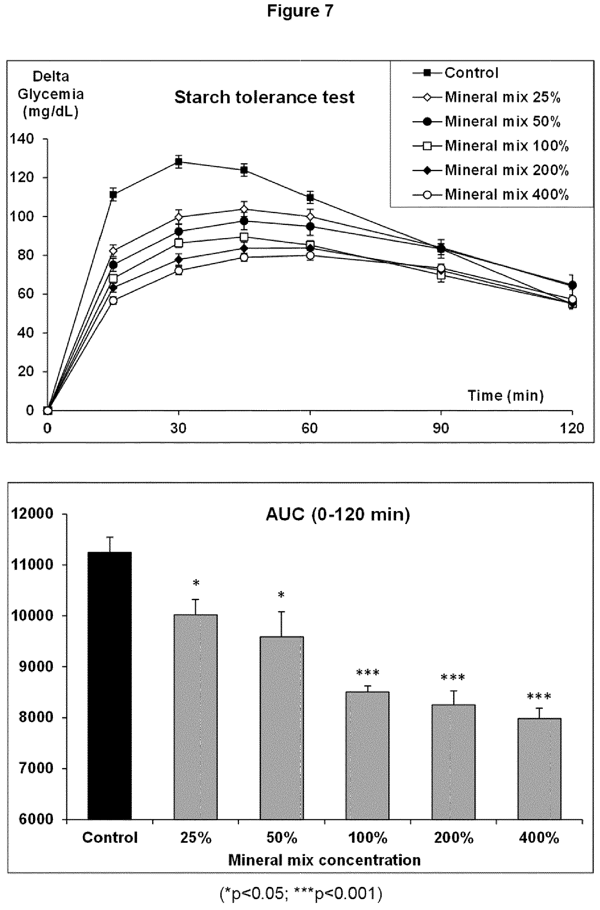

The figure 7 represents mean values +/- standard error of the mean (sem; n=8) and shows that the mix of minerals reduced the glycemic response to starch in a dose-dependent manner. The 100% dose of the mix of minerals was composed of zinc acetate 1 g/kg, selenomethionine 2.48 µg/kg, manganese citrate 0.7 mg/kg, copper acetate 0.114 mg/kg, and chromium picolinate 4.2 µg/kg of body weight. On the graph representing the area under the curve (AUC) between 0 and 120 min (Figure 6bis), the mix of minerals significantly reduced the glycemic response to starch from the lowest dose tested, i.e. 25% of the mineral mix (t-test p<0.05).

### Example 10: Combination of cinnamon procyanidin extract (CPE) and a mix of minerals on the glycemic response to starch in rat

The protocol was the same as for Examples 3 and 5.

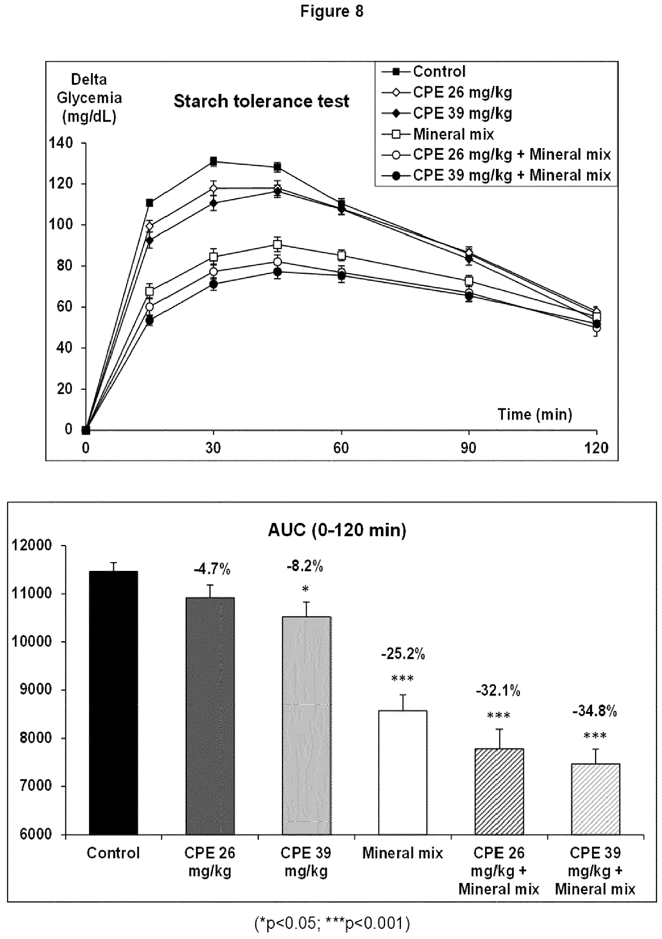

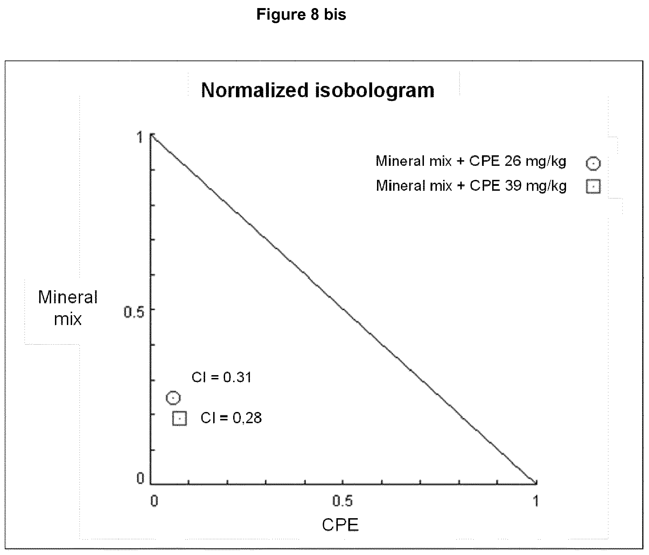

The figure 8 represents mean values +/- standard error of the mean (sem; n=8) and shows that the CPE reduced the glycemic response to starch by 4.7% at 26 mg/kg of body weight and by 8.2% at 39 mg/kg of body weight (t-test p<0.05), whereas a mineral mix composed of zinc acetate 1 g/kg, selenomethionine 2.48 µg/kg, manganese citrate 0.7 mg/kg, copper acetate 0.114 mg/kg, and chromium picolinate 4.2 µg/kg of body weight reduced the glycemic response to starch by 25.2% (t-test p<0.001). When the CPE and the mineral mix were given together, the effect reached 32.1 % (t-test p<0.001) and 34.8% (t-test p<0.001) for the combination with 26 and 39 mg/kg of body weight of CPE, respectively.

The isobole (shown on figure 8 bis) analysis revealed that the CPE and the mineral mix have a very strong synergistic effect on the glycemic response (Cl=0.31 and 0.28 for the combination with 26 and 39 mg/kg of body weight of CPE, respectively).

### Example 11: Combination of cinnamon procyanidin extract (CPE) and yeasts enriched in minerals on the glycemic response to starch in rat

The protocol was the same as for Example 3.

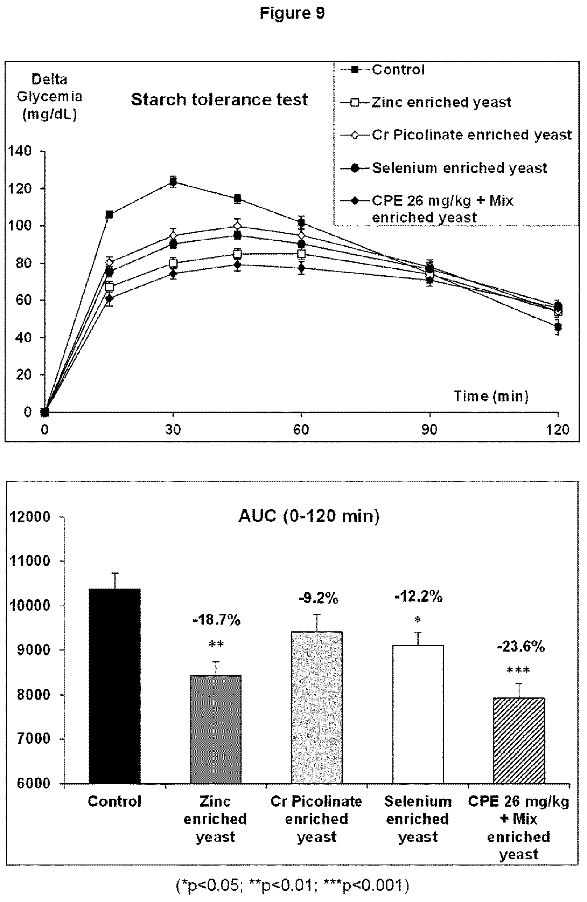

The figure 9 represents mean values +/- standard error of the mean (sem; n=8) and shows that yeasts enriched in minerals also reduced the glycemic response to starch. Zinc enriched yeast at 13.75 mg/kg of body weight (corresponding to 0.3 mg/kg of body weight of zinc element) reduced the glycemic response by 18.7% (t-test p<0.01), chromium picolinate enriched yeast at 0.25 mg/kg of body weight (corresponding to 0.5 µg/kg of body weight of chromium element) reduced the glycemic response by 9.2%, and selenium enriched yeast at 0.44 mg/kg of body weight (corresponding to 1 µg/kg of body weight of selenium element) reduced the glycemic response to starch by 12.2% (t-test p<0.05). When the CPE and a mix of the 3 mineral enriched yeasts (zinc, chromium picolinate, and selenium) were given together, the effect reached 23.6% (t-test p<0.001).

### Example 12: Combination of cinnamon procyanidin extract (CPE) and a mix of minerals on the glycemic response to glucose in rat

The protocol was the same as for Example 3 except that starch was replaced by glucose corresponding to an oral glucose tolerance test (OGTT). OGTT were conducted through the acute administration by oral gavage of a 20% glucose solution at 2 g/kg or 10 mL/kg of body weight containing the products to be tested.

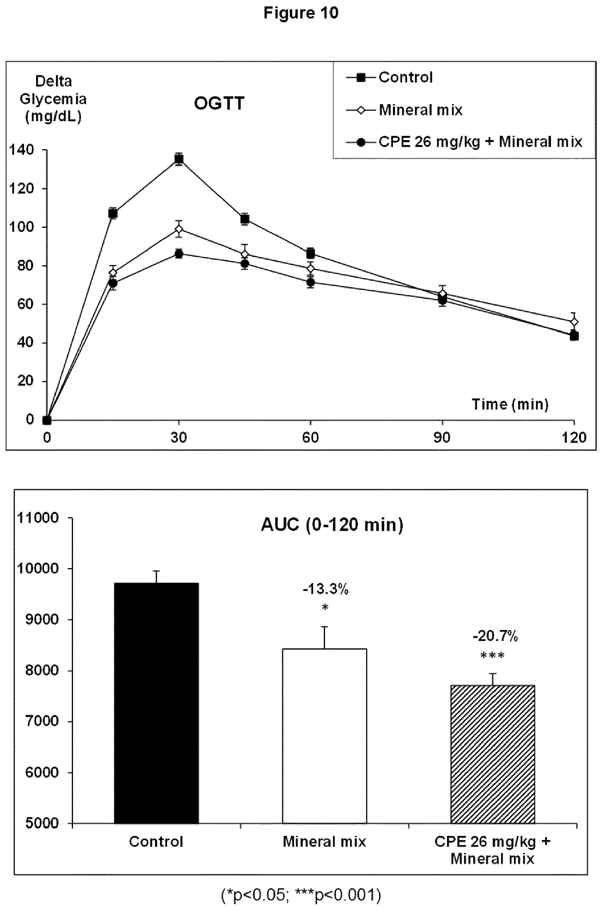

The figure 10 represents mean values +/- standard error of the mean (sem; n=8) and shows that a mineral mix composed of zinc acetate 1 g/kg, selenomethionine 2.48 µg/kg, manganese citrate 0.7 mg/kg, copper acetate 0.114 mg/kg, and chromium picolinate 4.2 µg/kg of body weight reduced the glycemic response to glucose by 13.3% (t-test p<0.05). When the CPE and the mineral mix were given together, the effect reached 20.7% (t-test p<0.001).

## Claims

1. Composition comprising :
- procyanidins and
- a mineral mix comprising chromium and at least one mineral selected in the group consisting of zinc, copper, manganese, selenium and magnesium.

2. Composition according to claim 1, **characterized in that** the mineral mix comprises chromium, zinc and at least one mineral selected in the group consisting of copper, manganese, selenium and magnesium.

3. Composition according to claim 1, **characterized in that** the mineral mix comprises chromium, zinc, copper and at least one mineral selected in the group consisting of manganese, selenium and magnesium.

4. Composition according to claim 1, **characterized in that** the mineral mix comprises chromium, zinc, copper, manganese and at least one mineral selected in the group consisting of selenium and magnesium.

5. Composition according to claim 1, **characterized in that** the mineral mix comprises chromium, zinc, copper, manganese, selenium and magnesium.

6. Composition according to any of the claims 1 to 5, for use in the prevention or treatment, by oral administration prior to or simultaneously with the consumption of a food product rich in carbohydrates, of glucose related metabolism disorder.

7. Composition according to claim 6, **characterized in that** the disorder is selected in the group consisting of pre-diabetes, diabetes, hyperinsulinemia, hyperglycemia, metabolic syndrome, overweight, obesity and combinations thereof.

8. Composition according to claim 7, **characterized in that**, the hyperglycemia is a post-prandial hyperglycemia.

9. Composition according to claim 6, **characterized in that** the oral administration is one, two or three times a day, prior to or simultaneously with the meals.

10. Composition according to claim 6, **characterized in that** the simultaneous oral administration comprises the incorporation of the composition in the food product prior to its consumption.

11. Nutritional composition or food product containing an effective amount of the composition according to any of claims 1 to 5.

12. Nutritional composition or food product according to claim 11 for its use in the prevention or treatment of glucose related metabolism disorder.

13. Nutritional composition or food product according to claim 12, **characterized in that** the disorder is selected in the group consisting of pre-diabetes, diabetes, hyperinsulinemia, hyperglycemia, metabolic syndrome, overweight, obesity and combinations thereof.

14. Nutritional composition or food product according to claim 11 for its use in the reduction of the glycemic index of the said nutritional composition or food product.

15. Nutritional composition or the food product according to claim 11 to 14 **characterized in that** it contains between 0.01 and 5% in weight of the nutritional composition or food product, particularly between 0.025 and 2.5%, more particularly between 0.05 and 1%, even more particularly between 0.1 and 0.5% of a composition according to any one of claims 1 to 5.
